# DEMANDE DE BREVET EUROPEEN

(11) **EP 4 040 780 A1**
(43) Date de publication de la demande: **10.08.2022**
(21) Numéro de dépôt: 22155665.7
(22) Date de dépôt: 08.02.2022
(51) Int. Cl.: H04N 5/378

(54) **DETECTEUR NUMERIQUE A INTEGRATION NUMERIQUE DE CHARGES**

(30) Priorité: 08.02.2021 FR 2101174
(71) Demandeur: Trixell, 38430 Moirans (FR)
(72) Inventeur: FAVRE, Xavier, 38430 MOIRANS (FR); SIAUD, Martin, 38430 MOIRANS (FR)
(74) Mandataire: Marks & Clerk France

(57) **Abrégé**

L'invention concerne un détecteur numérique (100) comprenant un capteur plan et un premier substrat monolithique et un deuxième substrat monolithique, le capteur plan (11) étant positionné sur le premier substrat monolithique, le détecteur comprenant sur le premier substrat monolithique :
a. un ensemble de pixels (P(i,j)) organisé en matrice (13) selon des lignes et des colonnes et configuré de façon à générer des charges en fonction d'un rayonnement;
b. des conducteurs de colonnes (Yj), chacun reliant les pixels (P(i,j)) d'une même colonne et destinés à transporter les charges générées par les pixels (P(i,j)) ;
sur le deuxième substrat monolithique :
c. pour chacun des conducteurs de colonnes (Yj), un préamplificateur de charges (20) relié au conducteur de colonne (Yj), formant un conducteur de colonnes préamplifié (21j) et destiné à intégrer les charges transportées par ledit conducteur de colonne (Yj);
d. au moins un convertisseur analogique/numérique (22j) relié en série aux conducteurs de colonnes préamplifiés (21j), destiné à convertir les charges intégrées en sortie des préamplificateurs de charges (20) en une tension numérique ;
e. un bloc sérialisateur (23) relié au au moins un convertisseur analogique/numérique (22j), destiné à générer une tension de sortie.

## Description

L'invention se situe dans le domaine technique de l'imagerie médicale, et plus précisément dans celui de la lecture d'une matrice de pixels passifs typiquement sur dalles de type aSi (silicium amorphe) ou IGZO (abréviation du terme anglo-saxon Indium Gallium Zinc Oxide). Elle concerne plus particulièrement un détecteur numérique et l'intégration des charges électriques provenant des pixels via les colonnes de la matrice de pixels afin de les convertir directement en signal numérique.

Dans le domaine de l'imagerie à rayons X, on distingue quatre éléments constitutifs :
a. la source de rayonnement qui émet les rayons X ;
b. les filtres qui modifient les propriétés du rayonnement suivant le besoin de l'application ;
c. l'objet à imager ;
d. le détecteur qui convertit les photons X en une image numérique.

L'invention concerne le détecteur numérique, et plus particulièrement la conversion de charges électriques reçues au niveau du pixel en signal électrique proportionnel à la quantité de charges reçues. Le détecteur numérique peut mettre en œuvre différentes technologies possibles. Une des technologies utilise un scintillateur permettant de convertir les rayons X en photons visibles et une matrice de photodiodes permettant de convertir les photons visibles en un signal électrique. Elle peut aussi être applicable avec des photo-conductances effectuant la conversion directe rayons X/électrons.

La qualité de l'image est directement liée au bruit de la chaine électronique dont une part importante vient de la capacité de la colonne de la matrice de pixels.

La plupart des systèmes actuels utilisent de simples circuits de conversion charge-tension analogique associés à des convertisseurs analogiques/numériques.

D'une part, les montages intégrateurs standards qui assurent la conversion charge-tension ne sont pas optimaux vis-à-vis du processus d'intégration et conversion de charges en tension. Ils sont généralement associés à un mécanisme de correction appelé double échantillonnage corrélé ou CDS (abréviation du terme anglo-saxon Correlated Double Sampling) nécessitant une seconde intégration à vide des charges présentes sur la colonne et nécessitant donc un temps supplémentaire opérationnel rédhibitoire en terme de vitesse d'exécution.

D'autre part, le convertisseur analogique/numérique en aval nécessite une chaîne image analogique permettant d'adapter le signal analogique de sortie en tension d'entrée du convertisseur analogique/numérique. Il en résulte des défauts liés à l'association des deux blocs. Autrefois externe, ce qui avait pour conséquence d'accumuler les effets parasites à chaque interface, la tendance est maintenant à l'intégration du convertisseur analogique/numérique au sein du même circuit, mais les deux fonctions restent dissociées avec des blocs de convertisseur analogique/numérique nécessitant une fonction de multiplexage.

Les solutions proposées dans l'art antérieur reposent essentiellement sur l'utilisation du CDS (double échantillonnage corrélé).

Les solutions qui ne comportent pas de préamplificateur nécessitent d'utiliser une correction avec un impact rédhibitoire sur le temps d'intégration puisqu'il faut réaliser une double lecture de la colonne pour supprimer les effets indésirables. En effet, une correction type CDS nécessite un reset de la colonne pour faire une lecture à vide de celle-ci qui est ensuite soustraite du signal. Ce reset génère un nouveau bruit kTC qu'il faut filtrer. Un filtrage passe-bas est utilisé pour réduire ce bruit colonne, néanmoins les constantes de temps nécessaires à un filtrage efficace ne sont pas compatibles avec les temps de convergence du pixel, d'où l'impact sur le temps de lecture de la matrice de pixels.

Dans le cas d'une lecture avec un préamplificateur de charges associé à une colonne, le composant peut intégrer les charges de la colonne pendant le temps minimal nécessaire pour transférer les charges du pixel vers la colonne.

Dans le cas d'une lecture en mode CDS avec un intégrateur sans préamplificateur de charges, des temps additionnels sont nécessaires pour remettre à niveau la colonne et échantillonner le signal avant l'intégration des charges de la colonne pendant le temps minimal nécessaire pour transférer les charges du pixel vers la colonne.

Autrement dit, les solutions de l'art antérieur proposent une conversion de charges en tension analogique dans la colonne. Puis un multiplexage des différentes colonnes est réalisé vers un bloc de conversion analogique/numérique. Les données analogiques sont multiplexées vers le convertisseur analogique/numérique. Le fait d'avoir un circuit ADC indépendant génère des bruits et des distorsions supplémentaires. Les documents US 5184018 A et US 6642494 B1 divulguent un détecteur matriciel de l'art antérieur. Dans les deux cas, le système utilise un circuit sans préamplificateur de charges en amont, entre le capteur composé de la matrice de pixels et la chaîne de traitement, avec multiplexeur analogique et ADC bloc, les ADC étant déportés hors substrat du circuit de traitement. Le document Simoni et al "A digital vision sensor" (XP027220184) divulgue un capteur comportant un convertisseur analogique/numérique par voie, ne comportant pas de préamplificateur, et visant à convertir le courant d'une photodiode embarquée générant des courants significativement importants.

Un ADC bloc ou monolithique nécessite un multiplexage des voies en entrée et son temps de conversion équivaut à un temps ligne divisé par autant de voies que la matrice de pixels (cela pouvant aller de 1000 à 5000 voies typiquement, voire plus), cela afin que le temps total de conversion soit transparent par rapport au temps de lecture de la matrice de pixels et enchaîner les lignes et les images sans latence. Un ADC parallèle intégré dans la colonne permet de réduire le temps de conversion à un seul temps ligne puisqu'il y a un ADC par voie de traitement. Il en résulte un design d'ADC totalement spécifique car en dehors de la résolution initiale qui reste identique, la vitesse de conversion est totalement différente dans les 2 cas et les contraintes de consommation et surface varient drastiquement.

A noter que la réalisation d'un tel ADC n'est pas évidente puisqu'on se trouve à la frontière de deux types de convertisseurs : lent à très haute résolution et rapide à faible résolution. Les caractéristiques demandées à l'ADC d'une telle application correspondent à un compromis entre une résolution moyenne associée à une vitesse importante.

L'invention vise à pallier tout ou partie des problèmes cités plus haut en proposant un détecteur numérique présentant une fusion d'un préamplificateur de charges et d'un convertisseur analogique/numérique au sein d'une voie de traitement unique, le tout à l'intérieur d'un même circuit intégré monolithique. Une telle association permet de réduire le bruit kTC induit par la colonne et de minimiser le bruit de la chaîne en aval. Le préamplificateur permet de réduire le bruit colonne spécifique dit kTC. L'intégration de l'ADC et son imbrication avec le convertisseur charge-tension permet quant à elle de réduire les bruits parasites en réduisant la chaîne de traitement et en évitant le multiplexage des données en entrée. Le résultat est transmis sous forme de tension numérique sérialisée garantissant ainsi l'absence de détérioration ultérieure du signal. L'invention permet l'intégration rapide des charges provenant de la matrice de pixels en générant un minimum de perturbations du signal.

Le challenge de la mise en place de l'invention par un ADC parallèle est d'arriver à intégrer un ADC qui est un bloc conséquent et de le fusionner avec le routage unitaire de la voie (colonne) : en plus de la complexité de la réalisation spécifique, la surface et la consommation deviennent critiques car il y a autant d'ADCs que de colonnes dans la matrice de pixels.

A cet effet, l'invention a pour objet un détecteur numérique comprenant un capteur plan, un premier substrat monolithique et un deuxième substrat monolithique, le capteur plan étant réalisé sur le premier substrat monolithique, le détecteur comprenant :
sur le premier substrat monolithique :
   - un ensemble de pixels organisé en matrice selon des lignes et des colonnes et configuré de façon à générer des charges en fonction d'un rayonnement frappant le détecteur ;
   - des conducteurs de colonnes, chacun reliant les pixels d'une même colonne et destinés à transporter les charges générées par les pixels ;
sur le deuxième substrat monolithique :
   - pour chacun des conducteurs de colonnes, un préamplificateur de charges relié au conducteur de colonne, formant un conducteur de colonnes préamplifié et destiné à intégrer les charges transportées par ledit conducteur de colonne;
   - au moins un convertisseur analogique/numérique relié en série aux conducteurs de colonnes préamplifiés, destiné à convertir les charges intégrées en sortie des préamplificateurs de charges en une tension numérique ;
   - un bloc sérialisateur relié au au moins un convertisseur analogique/numérique, destiné à générer une tension de sortie à partir des tensions numériques du au moins un convertisseur analogique/numérique.

Avantageusement, à chaque conducteur de colonnes préamplifié est relié à un parmi les au moins un convertisseur analogique/numérique.

Avantageusement, au moins deux des conducteurs de colonnes préamplifiés sont connectés ensemble, et les au moins deux conducteurs de colonnes préamplifiés connectés ensemble convergent vers un parmi les au moins un convertisseur analogique/numérique.

Avantageusement, le bloc sérialisateur est positionné sur le deuxième substrat monolithique, en aval du au moins un convertisseur analogique/numérique.

L'invention sera mieux comprise et d'autres avantages apparaîtront à la lecture de la description détaillée d'un mode de réalisation donné à titre d'exemple, description illustrée par le dessin joint dans lequel :
[Fig.1] La figure 1 représente schématiquement un détecteur d'images traditionnel;
[Fig.2] La figure 2 représente schématiquement un détecteur d'images de l'art antérieur et le détail d'une architecture d'intégration de charges d'un détecteur d'images de l'art antérieur ;
[Fig.3] La figure 3 représente schématiquement un détecteur d'images selon l'invention et le détail d'une architecture d'intégration de charges d'un détecteur d'images selon l'invention.

Sur ces figures, dans un souci de clarté, les échelles ne sont pas respectées. Par ailleurs, les mêmes éléments porteront les mêmes repères dans les différentes figures.

De manière générale, l'invention mentionne un détecteur d'images traditionnel comprenant typiquement un capteur plan comprenant un ensemble de pixels organisé en matrice selon des lignes et des colonnes, des blocs d'adressage de lignes, des blocs de lecture de colonnes, des conducteurs de ligne reliant les lignes de pixels à un bloc d'adressage de lignes, et des conducteurs de colonne reliant les colonnes de pixels à un bloc de lecture de colonnes. Il est à noter que, dans le cadre de la présente demande de brevet, les notions de colonne et de ligne n'ont qu'un sens relatif, une ligne de pixels et une colonne de pixels n'étant autres que des rangées de pixels disposées par exemple, et de manière non-limitative, perpendiculairement l'une à l'autre. Un conducteur de ligne, respectivement de colonne, est défini comme étant orienté parallèlement à une ligne de pixels, respectivement une colonne de pixels.

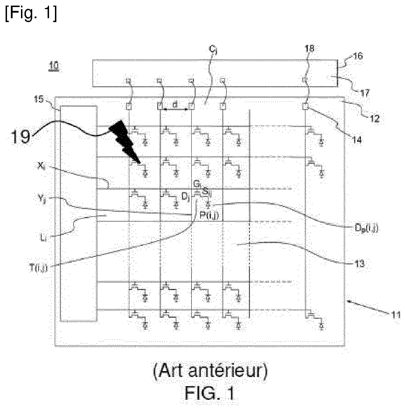

La figure 1 représente un détecteur d'images 10 traditionnel. Le détecteur d'images 10 comprend un capteur 11 réalisé sur un premier substrat monolithique 12. Le premier substrat monolithique 12 comprend un ensemble de pixels P(i,j) organisé en matrice 13 selon des lignes Li et des colonnes Cj. La matrice 13 peut comporter un nombre quelconque de lignes et de colonnes formant ainsi des pixels P(i,j). La matrice 13 forme une zone géographique sur le premier substrat 12. On note les pixels sous la forme générique P(i,j), où i et j sont des entiers naturels désignant respectivement le rang de la ligne et le rang de la colonne dans la matrice 13. L'ensemble des pixels P(i,j) est configuré de façon à générer des signaux en fonction d'un rayonnement frappant le détecteur 10. Le capteur 11 comprend des conducteurs de colonne Yj, chacun reliant les pixels d'une même colonne Cj. Les conducteurs de colonnes Yj sont destinés à transporter les signaux générés par les pixels P(i,j). De même, le capteur 11 comprend des conducteurs de ligne Xi, chacun reliant les pixels d'une même ligne Li. La matrice 13 de pixels P(i,j) comporte des colonnes Cj de rangs pairs et de rangs impairs. De même, la matrice 13 de pixels P(i,j) comporte des lignes Li de rangs pairs et de rangs impairs. Le capteur 10 comprend des plots de contact 14 situés à la périphérie du premier substrat 12 et en dehors de la matrice 13 de pixels P(i,j). Les plots de contact 14 sont reliés aux conducteurs de colonne Yj. Le détecteur d'images 10 comprend un bloc d'adressage de lignes 15 situé à proximité du premier substrat 12 et relié aux conducteurs de lignes Xi. On appelle bloc d'adressage de lignes 15 tout ensemble comportant au moins un bloc d'adressage de lignes. Le bloc 15 peut être intégré au premier substrat 12, comme représenté sur la figure 1, ou bien intégré à un substrat distinct. Le bloc d'adressage de lignes 15 permet d'adresser individuellement chaque ligne de pixels Li. Le détecteur d'images 10 comprend un bloc de lecture de colonnes 16 généralement réalisé sur un second substrat 17 différent du premier substrat 12. Le bloc de lecture de colonnes 16 comprend des points de connexion 18 reliant le bloc de lecture de colonnes 16 aux plots de contact 14. Le bloc de lecture de colonnes 16 permet de lire les signaux générés par les pixels de la ligne sélectionnée par le bloc d'adressage de lignes.

Un pixel P(i,j) comporte une photodiode Dp(i,j) associée à un interrupteur électronique T(i,j). Les photodiodes Dp(i,j) peuvent naturellement être remplacées par tout élément photosensible apte à générer un signal électrique lorsqu'il est soumis à un rayonnement de photons. La structure de pixel représentée sur la figure 1 est volontairement simplifiée et des structures plus complexes peuvent être mises en œuvre dans le cadre de l'invention.

L'interrupteur T(i,j) formé par un transistor est relié par sa grille Gi au conducteur de ligne Xi de la ligne i, par son drain Dj au conducteur de colonne Yj et par sa source Sij à la cathode de la photodiode Dp(i,j). Les anodes de toutes les photodiodes Dp(i,j) sont reliées à un potentiel commun, par exemple la masse. Le bloc d'adressage de ligne 15 comporte des éléments permettant de générer les signaux à injecter sur les conducteurs de ligne Xi pour piloter l'ouverture et la fermeture des transistors T(i,j). Le bloc de lecture de colonne 16 peut comporter des éléments permettant de traiter les signaux reçus sur les conducteurs de colonne Yj. En particulier, il peut s'agir d'un amplificateur et/ou d'un convertisseur analogique - numérique.

Traditionnellement, le capteur d'images 11 fonctionne de la manière suivante. Lors d'une phase de prise d'image, l'exposition des photodiodes Dp(i,j) à un rayonnement génère des charges électriques au niveau de la source Sij. La quantité de charges au niveau de chaque source Sij est fonction de l'intensité du rayonnement reçu par le pixel P(i,j) considéré. La phase de prise d'image est suivie d'une phase de lecture effectuée ligne par ligne. Les signaux injectés sur les différents conducteurs de ligne Xi passent successivement à l'état actif, de sorte que le potentiel de chaque conducteur de colonne Yj est successivement représentatif de la quantité de charges électriques accumulées dans les différents pixels P(i,j) de la colonne j.

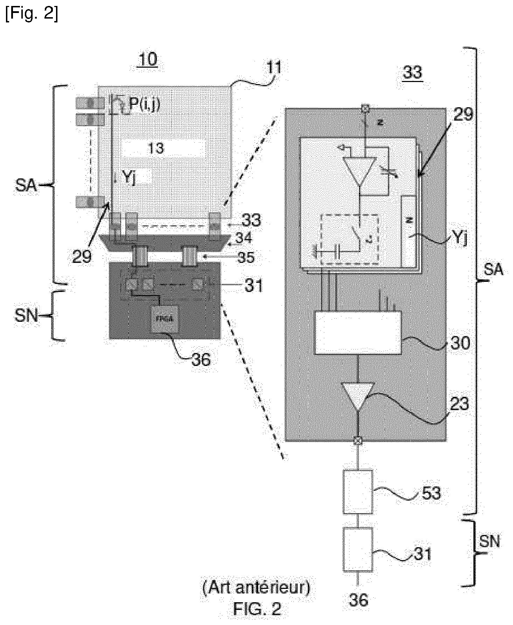

La figure 2 représente schématiquement un détecteur d'images 10 de l'art antérieur et le détail d'une architecture d'intégration de charges d'un détecteur d'images de l'art antérieur. Le détecteur numérique 10 de l'art antérieur comprend un capteur plan 11 réalisé sur un premier substrat monolithique. Le capteur plan 11 comprend un ensemble de pixels P(i,j) organisé en matrice 13 selon des lignes Li et des colonnes Cj et configuré de façon à générer des charges en fonction d'un rayonnement 19 frappant le détecteur 10. Le capteur 11 comprend des conducteurs de colonnes Yj, chacun reliant les pixels P(i,j) d'une même colonne Cj et destinés à transporter les charges générées par les pixels P(i,j). A ce stade, le signal est analogique. En sortie du capteur 11 se trouvent un ensemble de circuits intégrés de lecture 33 avec sortie analogique. Ces circuits 33 sont connectés à une carte fille 34, connectée par des connecteurs de carte 35 à des convertisseurs analogiques/numériques 31. Autrement dit, le signal est traité analogiquement en dehors du premier substrat. La zone du signal analogique est représentée par la référence SA. Le traitement numérique, représenté par la référence SN, a lieu après la sortie des convertisseurs analogiques/numériques 31.

L'intégration des charges en pied de colonne est décrite à l'aide du détail présenté sur la partie droite de la figure.

Dans cette architecture d'intégration de charges du détecteur 10 de l'art antérieur, chacune des N colonnes est reliée à un multiplexeur analogique 30, lui-même relié à un bloc convertisseur analogique/numérique 31. Autrement dit, les charges des N voies, c'est-à-dire N conducteurs de colonne Yj, sont intégrées et échantillonnées (29), puis multiplexées analogiquement vers un ou plusieurs blocs convertisseurs analogiques/numériques 31 externes. La conversion des données est faite à l'extérieur de l'architecture et nécessite une adaptation du signal (gain, buffering 53, etc). Pour chaque voie, un bloc sérialisateur 23 permet de sérialiser les signaux vers la sortie, par exemple vers un circuit FPGA 36. Enfin, une correction CDS est appliquée, c'est-à-dire que dans l'architecture de l'art antérieur, il y a une deuxième lecture avec un reset préalable de la colonne pour faire une lecture à vide de celle-ci qui est ensuite soustraite du signal.

Dans cette architecture de l'art antérieur, la conversion des charges en tension analogique est réalisée dans les colonnes. Ensuite, en dehors des colonnes, un multiplexage des différentes colonnes vers le bloc convertisseur analogique/numérique est réalisé. Les données analogiques sont multiplexées vers le convertisseur.

En plus de nécessiter une fonction de multiplexage, cette solution de l'art antérieur requiert un temps opérationnel supplémentaire pouvant s'avérer rédhibitoire en termes de vitesse d'exécution du fait du double échantillonnage corrélé. En outre, le traitement analogique du signal en aval de la colonne ne permet pas d'assurer l'intégrité des données.

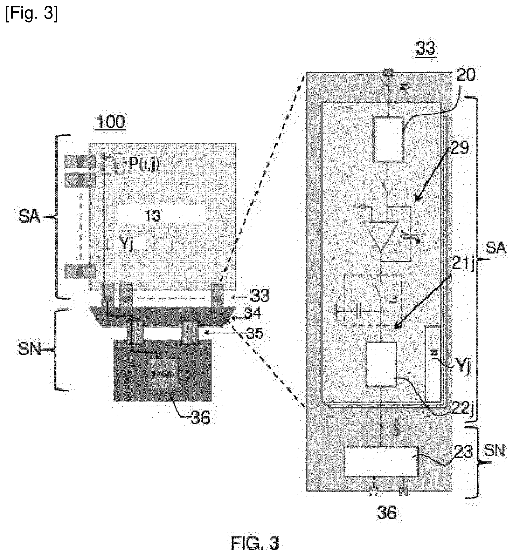

La figure 3 représente schématiquement un détecteur d'images 100 selon l'invention et le détail d'une architecture d'intégration de charges du détecteur d'images selon l'invention. La description de base du détecteur numérique 100 est similaire à celle du détecteur 10 de la figure 1. Les différences interviennent au niveau du bloc d'intégration présenté ici plus en détail sur la partie droite de la figure. Les références ci-après, même si elles ne sont pas toutes présentes sur la figure 3, sont reprises en se basant sur les éléments communs avec le détecteur de la figure 1 et de la figure 2 pour une meilleure compréhension. Le détecteur numérique 100 selon l'invention comprend un capteur plan 11 et un premier substrat monolithique et un deuxième substrat monolithique, le capteur plan 11 étant positionné sur le premier substrat monolithique. Le capteur plan 11 comprend un ensemble de pixels P(i,j) organisé en matrice 13 selon des lignes Li et des colonnes Cj et configuré de façon à générer des charges en fonction d'un rayonnement 19 frappant le détecteur 100. Le capteur 11 comprend des conducteurs de colonnes Yj, chacun reliant les pixels P(i,j) d'une même colonne Cj et destinés à transporter les charges générées par les pixels P(i,j). Selon l'invention, le détecteur comprend sur le deuxième substrat monolithique, distinct du premier substrat, pour chacun des conducteurs de colonnes Yj, un préamplificateur de charges 20 relié au conducteur de colonne Yj, formant un conducteur de colonnes préamplifié 21j et destiné à intégrer les charges transportées par ledit conducteur de colonne Yj. Le capteur 11 comprend au moins un convertisseur analogique/numérique 22j relié en série aux conducteurs de colonne préamplifiés 21j, destiné à convertir les charges intégrées en sortie des préamplificateurs de charges 20 en une tension numérique. Enfin, le capteur 11 comprend un bloc sérialisateur 23 relié au moins un convertisseur analogique/numérique 22j, destiné à générer une tension de sortie à partir des tensions numériques du au moins un convertisseur analogique/numérique 22j. Comme on le voit sur la figure 3, le signal est traité analogiquement sur le deuxième substrat monolithique. La zone du signal analogique est représentée par la référence SA. Le traitement numérique, représenté par la référence SN, a lieu dès la sortie du premier substrat monolithique.

Dans le détecteur numérique de l'invention, le bloc sérialisateur 23 est positionné sur le deuxième substrat monolithique, en aval du au moins un convertisseur analogique/numérique 22j.

La conversion analogique/numérique est réalisée sur chaque voie en parallèle avant la sérialisation sur une ou plusieurs sorties numériques, cette sérialisation pouvant être assimilée à un multiplexage numérique. Une particularité de l'invention réside dans la combinaison du pré-amplificateur de charges et du convertisseur analogique/numérique par voie en amont du bloc sérialisateur sur un seul substrat monolithique dissocié de la matrice de photodiodes.

Le principe de l'invention est ainsi basé sur un circuit monolithique qui assure la conversion de charges en tension numérique. Autrement dit, la numérisation a lieu dans chaque colonne, avant le multiplexage. L'invention permet ainsi de réaliser un multiplexage numérique moins sensible au bruit et aux perturbations des signaux analogiques. Ce multiplexage numérique est rendu possible par l'utilisation de convertisseurs sigma-delta dans chaque colonne. En d'autres termes, l'invention vise à convertir des rayons X en charges via un capteur spécifique puis d'amplifier cette charge (de l'ordre du pC) pour la numériser, par le biais d'un étage de préamplification et d'intégration.

L'invention permet une conversion à plus de 16 bits, sur des matrices typiquement de très grandes tailles (au-delà du million de pixels) pour un nombre de trames équivalent.

Contrairement à l'art antérieur qui propose une solution dans laquelle on réalise un multiplexage analogique suivi d'une numérisation, l'invention propose de positionner le convertisseur analogique/numérique sur chaque voie et de réaliser ensuite un multiplexage numérique. L'invention consiste à réaliser une fusion, ou imbrication, de l'ADC sur le deuxième substrat. Il en résulte un routage de la voie avec le reste des blocs.

Dans un premier mode de réalisation de l'invention, la solution consiste à associer pour chaque voie un préamplificateur de charges 20 et un convertisseur analogique/numérique (ADC) 22j dans un même circuit intégré monolithique. Autrement dit, à chaque conducteur de colonnes préamplifié 21j est relié un convertisseur analogique/numérique 22j.

L'intégration du convertisseur analogique/numérique sur le premier substrat et directement au conducteur de colonne préamplifié permet une réduction du nombre d'interfaces entre le domaine analogique et le domaine numérique. Cette solution permet la génération directe d'une ou plusieurs sorties numériques à partir de la première puce, sans risque de dégradation du signal après sa sortie. Enfin, cette intégration directe permet une simplification des cartes électroniques dans le traitement ultérieur du signal.

Ainsi, cette intégration permet une amélioration significative des performances de linéarité, de bruit et de diaphonie tout en réduisant le coût et l'encombrement des composants qui réalisent la fonction intégration/numérisation.

Dans un autre mode de réalisation de l'invention, chaque voie est associée à un préamplificateur de charges et un convertisseur analogique/numérique (ADC) 22j est associé à plusieurs voies dans un même circuit intégré monolithique. Autrement dit, un convertisseur analogique/numérique 22j est relié à plusieurs conducteurs de colonnes préamplifiés 21j. Cette variante partage le convertisseur analogique/numérique entre plusieurs voies dans un souci d'économie de surface et de réduction de la densité lorsque l'application le permet.

Dans une variante de l'invention, au moins deux des conducteurs de colonnes préamplifiés 21j (par exemple quatre conducteurs de colonnes) peuvent être connectés ensemble et les au moins deux conducteurs de colonnes préamplifiés 21j connectés ensemble convergent vers un parmi les au moins un convertisseur analogique/numérique 22j.

L'invention repose sur la réalisation d'un convertisseur de charges électriques provenant d'une matrice de pixels directement en une tension numérique sur le deuxième substrat monolithique. Comme on le voit sur la figure 3, en sortie du capteur, le signal est numérique, l'intégrité des données de ce signal est ainsi assurée.

L'invention se distingue de l'art antérieur par l'association, pour chaque voie ou au moins pour une pluralité de voies, d'un préamplificateur de charges et un convertisseur analogique/numérique (ADC) dans un même circuit intégré monolithique. Cette intégration permet une amélioration significative des performances de linéarité, de bruit et de diaphonie tout en réduisant le coût et l'encombrement des composants qui réalisent la fonction intégration/numérisation.

Plus précisément, le préamplificateur de charge pallie les limitations des étages d'intégration standard en permettant d'intégrer la charge au même rythme que le transfert de la charge du pixel via la colonne jusqu'à l'entrée du circuit intégrateur. Cette disposition présente deux avantages. Le premier avantage est l'absence de perte de temps : il y a un gain significatif en temps de traitement complet donc en vitesse de lecture de la dalle et temps de transfert (le temps de traitement des donnés est inférieur au temps d'intégration). Le deuxième avantage est une réduction significative du bruit lié à l'intégration de la charge d'entrée notamment à fort gain.

De plus, l'association directe du préamplificateur directement au convertisseur analogique/numérique ADC permet de se dispenser de l'étage traditionnel de multiplexage analogique entre un groupe de colonnes et son convertisseur unique.

L'intégration du convertisseur analogique/numérique ADC à l'intérieur de la voie de traitement permet d'avoir un convertisseur analogique/numérique ADC parfaitement conçu et adapté à la situation et ainsi de limiter tous les effets parasites. Les avantages de cette intégration sont multiples :
a. amélioration très significative de la linéarité intégrale d'ensemble ;
b. amélioration très significative de la linéarité spatiale (notamment voie à voie) ;
c. réduction significative des performances de bruit ;
d. limitation des effets de diaphonie verticale (crosstalk) voie-à-voie (chaque voie de traitement indépendant jusqu'à la numérisation finale) ;
e. réduction du coût de la solution globale grâce à l'intégration.

Des inconvénients sont toutefois à prendre en compte. Il s'agit notamment de la difficulté de développer et d'intégrer un convertisseur analogique/numérique ADC compatible avec des applications nécessitant une haute résolution (14-16bits) et de tels niveaux de performances (vitesse, linéarité, bruit). De plus, la solution proposée par l'invention nécessite l'augmentation de la surface de la puce et donc le coût de la puce. Enfin, la solution proposée par l'invention implique une augmentation de la consommation de la puce nécessitant une dissipation thermique appropriée.

L'invention permet de réduire le bruit kTC induit par la colonne grâce au montage spécifique du préamplificateur de charges imbriqué avec un convertisseur analogique-numérique (ADC) afin de minimiser le bruit de la chaîne en aval. Le résultat est transmis sous forme de tension numérique sérialisée (et il n'y a plus de détérioration du signal après cette étape).

L'invention permet de répondre au besoin qui est de pouvoir intégrer aussi rapidement que possible la charge provenant de la matrice de pixels en générant le moins possible de perturbations du signal (bruit/linéarité/fuites/diaphonie) ce que permet de faire le préamplificateur de charge en l'associant avec un ADC au sein d'un même bloc sur la même puce. L'ADC et la vitesse de transfert de la sortie numérique doivent également être suffisamment rapides au regard des caractéristiques de la matrice de pixels (temps de convergence du pixel notamment).

Dans le détecteur 100 de l'invention, le convertisseur analogique/numérique est placé à l'intérieur de la colonne. Il n'y a plus de multiplexeur dans cette architecture, ce qui permet un gain de temps. Le traitement analogique est réalisé dans la colonne et livre une tension numérique en sortie de colonne. Ici, la notion de colonne est à comprendre comme étant étendue au-delà de la colonne physique sur la matrice de photodiodes et comprenant les "voies" individuelles dans le circuit de conversion externe à la matrice. Cela élimine les problèmes de stabilité ou de pollution du signal. En sortie de colonne, le détecteur de l'invention permet un traitement du signal en numérique, ce qui assure l'intégrité de l'image de la donnée analogique provenant du capteur.

La particularité de l'invention repose sur la conversion directe de charges en signal numérique, sans altération du signal par des étapes et/ou étages intermédiaires. L'invention est basée sur le couplage du préamplificateur de charges avec un convertisseur analogique/numérique ADC dans la même voie de traitement d'une colonne de pixels.

Le fait de concevoir un convertisseur analogique/numérique et de l'utiliser en parallèle permet de convertir directement la charge en signal numérique sans autre étape intermédiaire (multiplexage analogique notamment) et donc de limiter les sources de bruit et de distorsions.

Associer le préamplificateur avec un tel convertisseur analogique/numérique ADC parallèle permet ainsi de créer un véritable convertisseur "charge-to-digital" (numériseur de charge), l'ensemble de la conversion se situant dans la voie de traitement de la colonne et ne demandant pas d'opérations supplémentaires sous forme analogique comme par exemple une opération de multiplexage.

Le convertisseur analogique/numérique ADC doit être parallèle (c'est-à-dire que nous avons un convertisseur analogique/numérique ADC par voie/préamplificateur ou alternativement par groupement de voies/préamplificateurs) et intégré à la chaîne immédiate de traitement de la colonne. Le convertisseur analogique/numérique ADC peut être de n'importe quel type (notamment approximations successives ou sigma-delta), la résolution est indifférente (en général 14 ou 16 bits, voire plus).

En fonction des exigences des applications visées (vitesse, bruit/linéarité, taille des pixels et possibilités de binning), et comme déjà mentionné, il est possible de considérer 1 convertisseur analogique/numérique parallèle commun pour plusieurs voies afin d'optimiser le design du circuit en termes de surface.

Le principe de l'invention peut s'appliquer à tous types d'imageurs avec des pixels passifs (imageur 1T), notamment les applications du type médical, NDT.

## Revendications

1. Détecteur numérique (100) comprenant un capteur plan (11) et un premier substrat monolithique et un deuxième substrat monolithique, le capteur plan (11) étant positionné sur le premier substrat monolithique, le détecteur (100) comprenant
sur le premier substrat monolithique :
a. un ensemble de pixels (P(i,j)) organisé en matrice (13) selon des lignes (Li) et des colonnes (Cj) et configuré de façon à générer des charges en fonction d'un rayonnement (19) frappant le détecteur (10) ;
b. des conducteurs de colonnes (Yj), chacun reliant les pixels (P(i,j)) d'une même colonne (Cj) et destinés à transporter les charges générées par les pixels (P(i,j)) ;
sur le deuxième substrat monolithique, distinct du premier substrat :
c. pour chacun des conducteurs de colonnes (Yj), un préamplificateur de charges (20) relié au conducteur de colonne (Yj), formant un conducteur de colonnes préamplifié (21j) et destiné à intégrer les charges transportées par ledit conducteur de colonne (Yj);
d. au moins un convertisseur analogique/numérique (22j) relié en série aux conducteurs de colonnes préamplifiés (21j), destiné à convertir les charges intégrées en sortie des préamplificateurs de charges (20) en une tension numérique ;
e. un bloc sérialisateur (23) relié au au moins un convertisseur analogique/numérique (22j), le au moins un convertisseur analogique/numérique (22j) étant disposé en amont du bloc sérialisateur (23) sur le deuxième substrat monolithique, le bloc sérialisateur (23) étant destiné à générer une tension de sortie à partir des tensions numériques du au moins un convertisseur analogique/numérique (22j).

2. Détecteur numérique (100) selon la revendication 1, dans lequel à chaque conducteur de colonnes préamplifié (21j) est relié un parmi les au moins un convertisseur analogique/numérique (22j).

3. Détecteur numérique (100) selon la revendication 1, dans lequel au moins deux des conducteurs de colonnes préamplifiés (21j) sont connectés ensemble, et les au moins deux conducteurs de colonnes préamplifiés (21j) connectés ensemble convergent vers un parmi les au moins un convertisseur analogique/numérique (22j).

4. Détecteur numérique (100) selon la revendication 1, dans lequel le bloc sérialisateur (23) est positionné sur le deuxième substrat monolithique, en aval du au moins un convertisseur analogique/numérique (22j).
